# EUROPEAN PATENT APPLICATION

(11) **EP 3 251 745 A1**
(43) Date of publication of application: **06.12.2017**
(21) Application number: 16742731.9
(22) Date of filing: 26.01.2016
(51) Int. Cl.: B01J 23/745, B01J 23/75, B01J 23/889, C10G 2/00

(54) **MONODISPERSE TRANSITION METAL NANO-CATALYST FOR FISCHER-TROPSCH SYNTHESIS AND PREPARATION METHOD THEREFOR AND APPLICATION THEREOF**

(30) Priority: 30.01.2015 CN 201510050801
(71) Applicant: Wuhan Kaidi Engineering Technology Research Institute Co., Ltd., Wuhan, Hubei 430223 (CN)
(72) Inventor: CHEN, Yilong, Wuhan Hubei 430212 (CN); ZHENG, Shenke, Wuhan Hubei 430212 (CN); CHEN, Jiangang, Wuhan Hubei 430212 (CN); SONG, Dechen, Wuhan Hubei 430212 (CN); ZHAN, Xiaodong, Wuhan Hubei 430212 (CN); ZHANG, Yanfeng, Wuhan Hubei 430212 (CN)
(74) Representative: Zeuner Summerer Stütz
(86) International application number: PCT/CN2016/072081
(87) International publication number: WO 2016/119669

(57) **Abstract**

A monodisperse transition metal nano-catalyst for Fischer-Tropsch synthesis and a preparation method therefor and an application thereof. The catalyst comprises transition metal. The transition metal is stably dispersed in an organic solvent in the form of monodisperse metal nanoparticles. The particle size of the transition metal is 1-100 nm. The specific surface area of the catalyst is 5-300 m²/g. The dispersity of the metal nanoparticles of the catalyst is high, and the catalyst can be directly applied to a Fischer-Tropsch synthesis reaction without filtering, cleaning, high-temperature roasting, and activated reduction.

## Description

### FIELD OF THE INVENTION

The invention relates to the field of Fischer-Tropsch synthesis catalyst, and more particularly to a monodisperse transition metal nano catalyst for Fischer-Tropsch synthesis, a preparation method and use thereof.

### BACKGROUND OF THE INVENTION

Fischer-Tropsch synthesis (F-T synthesis) is a reaction that converts synthesis gas prepared by gasifying carbon materials including coal, natural gas, and biomass to target product mainly composed by alkane and olefin. The target product barely contains sulfur, nitrogen, and aromatic compounds, thus the target product is processed to form environmental-friendly clean fuels. The F-T synthesis is an ideal way to fully utilize the carbon materials as a substitution for conventional fossil fuel. As the crude oil import in China increases, and the environmental requirement is enhanced, the F-T synthesis becomes increasingly important for energy security and environmental protection, and has attracted attention from researchers.

The performance of catalyst in the F-T synthesis is closely related to the chemical element composition. Elements of group VIII, such as iron, cobalt, nickel, and ruthenium have strong catalytic effect on the F-T synthesis, and are used as major metal components of the catalyst. The catalyst for industrial F-T synthesis mainly uses iron or cobalt as the major metal component and other metal elements as promotors so as to adjust and improve the performance of the catalyst.

Common methods for preparing the F-T synthesis catalyst are known to those skilled in the art: cobalt-based catalyst for F-T synthesis is usually prepared by impregnation method which loads the active metal components on a surface of oxide carrier; and iron-based catalyst for F-T synthesis is prepared by coprecipitation process or melting method. Catalysts prepared using different methods have obviously different performance in the F-T synthesis due to different microstructures of the catalysts. The pore structure inside the catalyst particles and the size distribution of metal particles are both crucial for the catalytic performance of the catalyst. Catalyst prepared using the impregnation method and the coprecipitation process is rich in pore structure, therefore, the catalytic performance of active metal in the pores is influenced by the concentration of raw materials and the internal diffusion. In addition, when the active components of metal particles are fitted in the carrier surface, the specific surface area of exposed active component is relatively small, which limits the catalytic performance of the catalyst. Meanwhile, researches indicate that when the particle size of the metal particles is controlled within a specific range, the catalytic activity and the product selectively of the catalyst when used in the F-T synthesis is the best, however, due to limitations of conventional preparation methods, the particle size of metal particles at the catalyst surface is difficult to control.

In view of the above problems, to improve the catalytic performance, researchers turn to no-load nano-particle catalyst, however, the nano-particle catalyst has disadvantages as low applicable temperature, low space time yield, and oversized active metal components. For example, Chinese patent CN 200710099011 disclosed a method for performing F-T synthesis and a special catalyst for the F-T synthesize. The patent mixed transition metal salt (iron, cobalt, nickel, ruthenium, rhodium, or a mixture thereof) with high-molecular stabilizer (polyvinylpyrrolidone or (BVIMPVP)Cl) to form a reaction mixture, and dispersed the reaction mixture in a liquid medium. The reaction mixture was reduced using hydrogen at a temperature between 100 and 200°C to yield a transition metal catalyst with between 1 and 10 nm of nano particles. The transition metal catalyst is used for F-T synthesis at a temperature between 100 and 200°C. However, the nano particle concentration of the catalyst prepared by the method is relatively low, and the highest concentration of transition metal salt in the liquid medium is only 0.014 mol/L. In addition, the patent uses high-molecular compound as the stabilizer, and the catalyst can only be applied to the F-T synthesis performed at a temperature lower than 200°C, moreover, the space time yield is relatively low, all these limits the industrial application of the catalyst. Chinese patent CN200810055122 disclosed a catalyst applicable in slurry bed reactor, a method for preparing the catalyst, and an application of the catalyst. The patent dissolved nitrate of transition metal (iron, cobalt, or nickel) in a C₆-C₈ straight chain alcohol solution and heated to reflux the solution so as to yield transition metal catalyst with between 5 and 200 nm of nano particles. The transition metal catalyst is used for F-T synthesis in the slurry bed reactor after being reduced and activated. However, due to the crystal water in the nitrate, on the one hand, the hydrogen bond on the metal particle surface is increased and the aggregation of crystal particles is enhanced (T. He, D. Chen, X. Jiao, Controlled Synthesis of Co3O4 Nanoparticles through Oriented Aggregation, Chem. Mater., 16 (2004) 737-743), on the other hand, the decomposition temperature of the cobalt nitrate is increased, resulting in fast formation and growth of metal crystal nucleus, both of which lead to final large-size aggregated particles (Li Zezhuang, Chen Jiangang, Wang Yuelun, Sun Yuhan, Preparation of Monodispersed Co/SiO2 Catalyst and Their Performance for Fischer-Tropsch Synthesis (J), Industrial Catalysis 2009, Vol. 17(9), 43-47).

### SUMMARY OF THE INVENTION

In view of the above-described problems, it is one objective of the invention to provide a monodisperse transition metal nano catalyst for Fischer-Tropsch synthesis, a preparation method and use thereof. The catalyst has high catalyst activity and the grain size of the active metal of the catalyst is controllable.

To achieve the above objective, in accordance with one embodiment of the invention, there is provided a monodisperse transition metal nano catalyst for Fischer-Tropsch synthesis, comprising a transition metal and an organic solvent. The transition metal is stably dispersed in the organic solvent in the form of monodisperse nanoparticles; the transition metal has a grain size of between 1 and 100 nm; and the catalyst has a specific surface area of 5 and 300 m²/g.

In a class of this embodiment, the transition metal is manganese, iron, cobalt, ruthenium, or a mixture thereof.

In a class of this embodiment, the organic solvent is benzyl ether, aromatic alcohol, pyrrolidone, or liquid paraffin.

In a class of this embodiment, the grain size of the transition metal is between 5 and 20 nm.

In another aspect, the present disclosure also provides a method for preparing the monodisperse transition metal nano catalyst for Fischer-Tropsch synthesis, the method comprising:
1) dissolving an organic salt of the transition metal into the organic solvent comprising a polyhydric alcohol, to yield a mixture; and
2) heating and stirring the mixture in the presence of air or inert gas, holding the mixture at a temperature of between 150 and 250 °C for between 30 and 240 min, to yield the monodisperse transition metal nano catalyst for Fischer-Tropsch synthesis.

In a class of this embodiment, in 1), the transition metal is manganese, iron, cobalt, ruthenium, or a mixture thereof, and the organic salt is oxalate, acetylacetonate, or carbonyl metal salt.

In a class of this embodiment, in 1), the polyhydric alcohol is C₃-C₁₈ dihydric alcohol or trihydric alcohol, and the organic solvent is benzyl ether, aromatic alcohol, pyrrolidone, or liquid paraffin.

In a class of this embodiment, in 1), a molar ratio of the polyhydric alcohol to the organic salt of the transition metal is between 1-5: 1, and a molar ratio of the organic solvent to the organic salt of the transition metal is between 30-500: 1.

In a class of this embodiment, in 2), a heating rate of the mixture is between 1 and 10 °C /min, and a holding time of the temperature is between 60 and 120 min.

The present disclosure further provides a method for Fischer-Tropsch synthesis comprising applying the monodisperse transition metal nano catalyst of claim 1, the method comprising, without filtration, separation, cleaning, high temperature roasting and activation reduction, directly employing the catalyst for the Fischer-Tropsch synthesis, and controlling a reaction temperature of between 180 and 300 °C, a reaction pressure of between 1 and 3 megapascal, a feed volume ratio of hydrogen to carbon monoxide of between 1 and 2.5, and a total space velocity of between 0.5 and 15 L/h/g catalyst.

Advantages of the monodisperse transition metal nano catalyst for Fischer-Tropsch synthesis according to embodiments of the invention are summarized as follows:

First, the catalyst of the invention is a non-loaded nano metal particle catalyst, the nano metal particle can move freely in the reaction process, no need to attach to the surface of the carrier, thus increasing the specific surface area and enhancing the catalytic properties of the catalyst. In addition, the nano metal particles have high concentration.

Second, the grain size of the active component particles is adjustable, so the size of the metal nanoparticles is controllable.

Third, the preparation method of the invention is simple and easy to operate, environment friendly, can adjust the grain size of the metal nanoparticles, and the active component is stably dispersed in the organic solvent in the form of monodisperse nanoparticles, the disperse solvent is recyclable.

Fourth, the nano metal particles of the catalyst have high dispersity, in a slurry reactor, without involving filtration, separation, cleaning, high temperature roasting and activation reduction, the catalyst can be directly used for Fischer-Tropsch synthesis, and exhibits excellent catalytic properties and product selectivity.

### BRIEF DESCRIPTION OF THE DRAWINGS

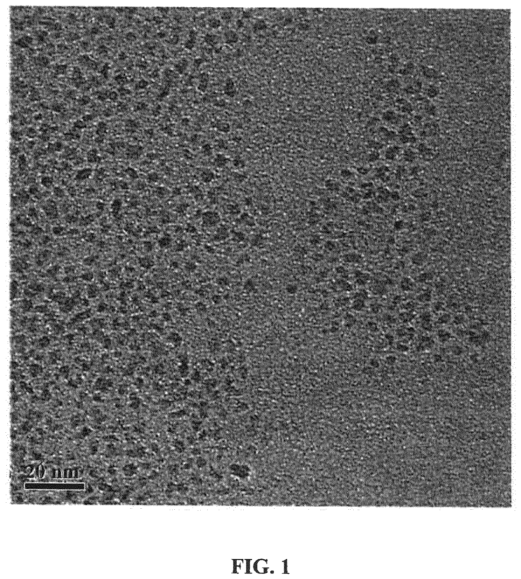

FIG. 1 is an image of a monodisperse transition metal nano catalyst for Fischer-Tropsch synthesis under transmission electron microscope in Example 1 of the invention.

### DETAILED DESCRIPTION OF THE EMBODIMENTS

For further illustrating the invention, experiments detailing a monodisperse transition metal nano catalyst for Fischer-Tropsch synthesis, a preparation method and use thereof are described hereinbelow combined with the drawings. It should be noted that the following examples are intended to describe and not to limit the invention.

### Example 1

6 g of ferric acetylacetonate (III) was dissolved in 550 mL of 2-pyrrolidone solution (with density of 1.116 g/mL), followed by addition of 3.5 g of 1,2-dihydroxydodecane, to yield a mixture. Thereafter, in the presence of mechanical stirring and air, the solution was heated to the temperature of 160 °C with a heating rate of 1°C/min. The solution was held for 120 min at the temperature of 160°C, and then cooled to room temperature, to yield a grey black nano iron colloid solution, which was sealed using 250 mL of liquid paraffin for use.

The prepared grey black nano iron colloid solution, together with the liquid paraffin, was transferred to a slurry bed reactor for Fischer-Tropsch synthesis immediately. The reaction temperature was 260°C, the feed volume ratio of hydrogen to carbon monoxide was 1.2, the gas space velocity was 13.7 L/h/g catalyst (the gas flow velocity was 13 L/h), and the reaction pressure was 2 MPa. Under the conditions, the performance evaluation of the catalyst is listed in Table **1,** and the microstructure of the catalyst is shown in FIG. **1****.**

### Example 2

2.6 g of cobalt oxalate (II) and 0.01 g of ruthenium nitrosyl nitrate (III) was dissolved in 250 mL of dibenzyl ether solution (with density of 1.04 g/mL), followed by addition of 10 g of 1,2-hexadecanediol, to yield a mixture. Thereafter, in the presence of mechanical stirring and argon gas, the solution was heated to the temperature of 250°C with a heating rate of 10°C /min. The solution was held for 80 min at the temperature of 250°C, and then cooled to room temperature, to yield a dark purple nano cobalt colloid solution, which was sealed using 250 mL of liquid paraffin for use.

The prepared dark purple nano cobalt colloid solution, together with the liquid paraffin, was transferred to a slurry bed reactor for Fischer-Tropsch synthesis immediately. The reaction temperature was 180°C, the feed volume ratio of hydrogen to carbon monoxide was 2.4, the gas space velocity was 4.8 L/h/g catalyst (the gas flow velocity was 5 L/h), and the reaction pressure was 3 MPa. Under the conditions, the performance evaluation of the catalyst is listed in Table **1.**

### Example 3

4 g of ferric acetylacetonate (III) and 2 g of cobalt acetylacetonate (II) was dissolved in 450 mL of benzyl alcohol solution (with density of 1.04 g/mL), followed by addition of 9 g of 1,2,4-butanetriol, to yield a mixture. Thereafter, in the presence of mechanical stirring and air, the solution was heated to the temperature of 200°C with a heating rate of 5°C /min. The solution was held for 60 min at the temperature of 200°C, and then cooled to room temperature, to yield a dark grey nano ferrocobalt colloid solution, which was sealed using 250 mL of liquid paraffin for use.

The prepared dark grey nano ferrocobalt colloid solution, together with the liquid paraffin, was transferred to a slurry bed reactor for Fischer-Tropsch synthesis immediately. The reaction temperature was 200°C, the feed volume ratio of hydrogen to carbon monoxide was 2, the gas space velocity was 7.3 L/h/g catalyst (the gas flow velocity was 8 L/h), and the reaction pressure was 1 MPa. Under the conditions, the performance evaluation of the catalyst is listed in Table **1.**

### Example 4

3.1 g of pentacarbonyl iron and 2.6 g of decacarbonyldimanganese were dissolved in 250 mL of liquid paraffin (with density of 0.87 g/mL), followed by addition of 5 g of 1,2,8-octanetriol, to yield a mixture. Thereafter, in the presence of mechanical stirring and nitrogen, the solution was heated to the temperature of 235°C with a heating rate of 8°C /min. The solution was held for 100 min at the temperature of 235°C, and then cooled to room temperature, to yield a grey black nano ferrimanganic colloid solution, which was sealed for use.

The prepared grey black nano ferrimanganic colloid solution was transferred to a slurry bed reactor for Fischer-Tropsch synthesis immediately. The reaction temperature was 240°C, the feed volume ratio of hydrogen to carbon monoxide was 1.8, the gas space velocity was 0.8 L/h/g catalyst (the gas flow velocity was 1 L/h), and the reaction pressure was 2 MPa. Under the conditions, the performance evaluation of the catalyst is listed in Table **1.**

**Table 1**

| Parameters | | Catalysts of the invention | | | |
|---|---|---|---|---|---|
| | | Example 1 | Example 2 | Example 3 | Example 4 |
| Physico-chemical properties | Average grain size of metal crystal (nm) | 5.3 | 47.0 | 18.7 | 83.3 |
| | Specific surface area of catalysts (m²/g) | 288.3 | 17.4 | 54.1 | 8.6 |
| Evaluation index | Reaction temperature (°C) | 260 | 180 | 200 | 240 |
| | Reaction pressure (MPa) | 2 | 3 | 1 | 2 |
| | Feed volume ratio of Hydrogen to carbon monoxide | 1.2 | 2.4 | 2 | 1.8 |
| | Space velocity (L/h/g catalyst) | 13.7 | 4.8 | 7.3 | 0.8 |
| Catalytic properties | CO conversion (%) | 73.2 | 26.7 | 33.1 | 32.8 |
| | Methane selectivity (mol %) | 3.2 | 7.7 | 6.1 | 2.8 |
| | Carbon dioxide selectivity (mol %) | 21.4 | 0.5 | 4.2 | 26.4 |
| | C₂₋₄ hydrocarbon selectivity (mol %) | 23.6 | 16.3 | 19 | 22.9 |
| | C₅+ hydrocarbon selectivity (mol %) | 51.8 | 75.5 | 70.7 | 47.9 |
| The data in the table was obtained by statistical analysis of images from transmission electron microscopy. | | | | | |

Based on the physico-chemical properties and catalytic properties of the catalyst as shown in Table 1, the preparation method of the present disclosure can quickly produce high activity metal nanometer particle catalyst with different grain sizes. In general, the smaller the grain size of the catalyst, the bigger the active specific surface area, and the higher the catalytic activity. However, the stability of the catalyst will decrease. The nano-metal catalyst having the grain size of 5-20 nm exhibits better comprehensive properties. Compared with conventional industrial catalysts, the catalyst of the invention exhibits better catalytic activity, lower methane selectivity, higher selectivity for C₂₋₄ hydrocarbons, so the catalyst of the invention has better application prospect.

## Claims

1. A monodisperse transition metal nano catalyst for Fischer-Tropsch synthesis, comprising:
a transition metal; and
an organic solvent;
wherein
the transition metal is stably dispersed in the organic solvent in the form of monodisperse nanoparticles;
the transition metal has a grain size of between 1 and 100 nm; and
the catalyst has a specific surface area of 5 and 300 m²/g.

2. The catalyst of claim 1, wherein the transition metal is manganese, iron, cobalt, ruthenium, or a mixture thereof.

3. The catalyst of claim 1 or 2, wherein the organic solvent is benzyl ether, aromatic alcohol, pyrrolidone, or liquid paraffin.

4. The catalyst of claim 1 or 2, wherein the grain size of the transition metal is between 5 and 20 nm.

5. A method for preparing the monodisperse transition metal nano catalyst for Fischer-Tropsch synthesis of claim 1, comprising:
1) dissolving an organic salt of the transition metal into the organic solvent comprising a polyhydric alcohol, to yield a mixture; and
2) heating and stirring the mixture in the presence of air or inert gas, holding the mixture at a temperature of between 150 and 250 °C for between 30 and 240 min, to yield the monodisperse transition metal nano catalyst for Fischer-Tropsch synthesis.

6. The method of claim 5, wherein in 1), the transition metal is manganese, iron, cobalt, ruthenium, or a mixture thereof, and the organic salt is oxalate, acetylacetonate, or carbonyl metal salt.

7. The method of claim 5 or 6, wherein in 1), the polyhydric alcohol is C₃-C₁₈ dihydric alcohol or trihydric alcohol, and the organic solvent is benzyl ether, aromatic alcohol, pyrrolidone, or liquid paraffin.

8. The method of claim 5 or 6, wherein in 1), a molar ratio of the polyhydric alcohol to the organic salt of the transition metal is between 1-5: 1, and a molar ratio of the organic solvent to the organic salt of the transition metal is between 30-500: 1.

9. The method of claim 5 or 6, wherein in 2), a heating rate of the mixture is between 1 and 10°C /min, and a holding time of the temperature is between 60 and 120 min.

10. A method for Fischer-Tropsch synthesis comprising applying the monodisperse transition metal nano catalyst of claim 1, the method comprising, without filtration, separation, cleaning, high temperature roasting and activation reduction, directly employing the catalyst for the Fischer-Tropsch synthesis, and controlling a reaction temperature of between 180 and 300 °C, a reaction pressure of between 1 and 3 megapascal, a feed volume ratio of hydrogen to carbon monoxide of between 1 and 2.5, and a total space velocity of between 0.5 and 15 L/h/g catalyst.
